# EUROPEAN PATENT SPECIFICATION

(11) **EP 2 007 470 B1**
(45) Date of publication and mention of the grant of the patent: **23.01.2013**
(21) Application number: 07755144.8
(22) Date of filing: 11.04.2007
(51) Int. Cl.: A61M 35/00, A61L 15/46

(54) **MULTI-LAYER WOUND DRESSINGS**
MEHRSCHICHTIGER WUNDVERBAND
PANSEMENTS MULTICOUCHES

(30) Priority: 11.04.2006 US 790813 P; 11.04.2006 US 790814 P; 09.03.2007 US 716008
(43) Date of publication of application: 31.12.2008
(73) Proprietor: Covidien LP, Mansfield, MA 02048 (US)
(72) Inventor: PATEL, Harish A., Norfolk, MA 02056 (US); SWANIKER, Hansen P., Bristol, RI 02809 (US); HEAGLE, David G., Taunton, Massachusetts, MA 02780 (US); WARD, Kate, Marshfield, MA 02050 (US); TRANCHEMONTAGNE, Alain, Warwick, RI 02889 (US)
(74) Representative: Gray, James
(86) International application number: PCT/US2007/008772
(87) International publication number: WO 2007/120617

(56) References cited:
- US-A- 5 931 800
- US-A1- 2004 047 763
- US-A1- 2004 082 925

## Description

### FIELD

The present invention is directed to wound dressings that are formed of multiple layers.

### BACKGROUND

In the discussion that follows, reference is made to certain structures and/or methods. However, the following references should not be construed as an admission that these structures and/or methods constitute prior art. Applicants expressly reserve the right to demonstrate that such structures and/or methods do not qualify as prior art.

Access to affordable health care is one of the most important issues facing the United States, as well as other countries around the world. One common technique for reducing the cost of providing health care services is to reduce the amount of time of in-patient hospital stays, and to reduce to a minimum the amount of person-to-person interaction between a patient and healthcare professionals. A related problem is that of the incidence of bacterial infection of post operative and other wounds. Such infections not only increase the demands on the resources of our healthcare system through lengthened hospital stays, but also require treatment with antibiotics. Due to the misuse of antibiotics, finding an effective treatment for such infections can prove difficult. Moreover, tremendous resources are needed to constantly develop new antibiotics to replace other such drugs that have been rendered ineffective.

In the area of wound care, a variety of wound dressings have been suggested. However, such wound dressings possess various deficiencies and shortcomings.

For example, a number of wound dressings have been proposed which include various anti-microbial agents. U.S. Patent Number 6,369,289 discloses a cellulosic dressing material having a calculated amount of PHMB applied thereto.

U.S. Patent Application Publication Number 2004/0082925 discloses a dressing comprising an inner layer of substantially hydrophilic material and an outer layer of substantially hydrophobic material on either side of the inner layer, and an anti-microbial agent contained therein.

U.S. Patent Number 4,655,756 discloses an article comprising a non-woven material treated with PHMB at a concentration ranging from 500 to 5000 ppm.

U.S. Patent Number 5,098,417 relates to a wound dressing constructed for the controlled release of an active agent into the wound.

Abstracted Chinese patent publication number CN1170564 A discloses a wound dressing comprising a zinc-calcium alginate in the form of a nonwoven fabric.

U.S. Patent Number 5,931,800 discloses a wound dressing including zinc and/or various alginates.

U.S. Patent Number 5,238,685 discloses a wound dressing comprising a mixed salt alginate, possibly in the form of calcium alginate fibers, and an anti-microbial agent.

U.S. Patent Number 5,759,570 discloses a multilayer wound dressing wherein the wound contacting layer thereof comprises a bioabsorbable and hydrophilic polymeric material.

U.S. Patent Number 6,599,525 discloses a dressing having a first skin-facing surface, and a discontinuous coating of a semi-solid composition having an ointment-like feel overlying a portion of the first surface.

U.S. Patent Number 4,699,792 discloses a self-adhesive medicinal plaster comprising a plurality of active ingredient elements spaced from each other and disposed on a carrier web.

U.S. Patent Number 4,643,180 discloses a surgical dressing having an adhesive, and wherein PHMB is provided in the adhesive at a concentration on the order of 1 to 20% by weight.

U.S. Patent Application Publication Number 2002/0022660 discloses a deep-penetrating anti-microbial composition comprising anti-microbial components and a combination of surfactants that do not include anionic.surfactants.

U.S. Patent Application Publication Number 2004/0047763 discloses an antimicrobial water-based system formulated to disinfect a catheter, etc., which includes approximately 10 to 200 mg of tetrasodium EDTA for each milliliter of water contained in the system.

U.S. Patent Application Publication Number 2004/0028722 discloses a wound dressing comprising a microbial-derived cellulose dressing material containing PHMB at a concentration on the order of 2700-7900 ppm.

U.S. Patent Application Publication Number 2004/0142019.discloses a microbial-derived cellulose wound dressing provided in the form of a hydrogel which may also contain PHMB and other additives.

U.S. Patent Application Publication Number 2005/0019380 discloses a wound dressing formed from a microbial-derived cellulose capable of donating liquid to a dry substrate, as well as absorbing exudate from a wound. The dressing may be treated to also contain PHMB.

U.S. Patent Number 3,797,494 discloses a bandage for use in the continuous administration of drugs to the skin or mucosa which includes a reservoir that can comprise a distinct layer containing a plurality of microcapsules and a drug release rate controlling microporous membrane material which meters the flow of drug transfer to the skin.

U.S. Patent Number 3,731,683 describes the bandage for the topical administration of therapeutically effective quantities of a topically active substance. The topically active substance is confined within a wall member which acts to control the release rate of drugs through the wall and into the skin.

U.S. Patent Number 3,598,122 discloses a bandage for the continuous administration of a systematically active drug via absorption through the skin or oral mucosa comprising a backing member and a reservoir having a wall distant from the backing member, the wall being permeable so as to permit passage of the drug in a controlled manner for absorption through the skin.

U.S. Patent Application Publication Number 2005/0048139 discloses compositions which may include an anti-microbial agent as well as a zinc-containing compound, which reportedly serves to prevent irritation of the skin.

Despite the above, a need exists in the art for a wound dressing which facilitates the economical and effective delivery of health care services in the wound care area. Thus, a need exists in the art for wound dressings which have increased effectiveness by speeding the wound-healing process, and provide enhanced capabilities for preventing infection. A need also exists for wound dressings that retain their wound-healing capabilities for extended periods of time, thereby requiring less frequent changing thus minimizing the amount of person-to-person contact necessary between a patient and healthcare professionals.
US 2004/082925 discloses a medical dressing containing an anti-microbial agent. The medical dressing comprises a layered fabric comprising an inner layer of substantially hydrophilic material, an outer layer of substantially hydrophobic material on both sides of the inner layer and an anti-microbial agent. The anti-microbial agent is contained within the inner layer.

### SUMMARY

According to certain aspects of the present invention, the present invention includes, but is not limited to, a wound dressing which is constructed to accelerate the wound-healing process. According to an additional optional aspect, the present invention includes, but is not limited to, a wound dressing which is constructed such that it retains its wound-healing properties for an extended period of time, and thus does not have to be changed as frequently as conventionally-constructed wound dressings. According to another aspect the present invention includes, but is not limited to, a wound dressing that possesses increased effectiveness in preventing infection.

The present invention includes, but is not limited to, two general approaches for achieving the above-stated optional objectives. First, a wound dressing can be provided with a combination of additives which, when provided in a wound dressing according to the teachings contained herein serve to increase the effectiveness of the wound dressing to promote healing relative to conventionally-constructed wound dressing materials containing conventional anti-microbial agents. Second, a wound dressing can be provided which generally contains a higher degree of anti-microbial agent, such as PHMB, than is typically contained in comparable wound dressings. Through specific wound dressing constructions and targeted and/or controlled release of anti-microbial and other agents, the wound dressing can increase its effectiveness over an extended period of time relative to conventional wound dressing constructions and compositions.

Consistent with the above, according to one optional aspect of the present invention, increased control of bioburdens is provided, without necessarily resorting to increased concentrations of anti-microbial agents, such as PHMB. According to a further optional aspect of the present invention, the wound dressing is provided which reduces the risk of infection, or facilitates the control of an existing infection, without change to the existing wound care protocol. According to yet a further optional aspect of the present invention, there is provided a wound dressing which will effectively increase the spectrum of activity of the anti-microbial agent contained therein. According to another optional aspect of the present invention, a wound dressing is provided which provides targeted and/or controlled delivery of an anti-microbial agent and/or additional additives contained in the wound dressing to the wound site. According to yet another optional aspect, the dressing of the present invention promotes migration of microbes from the wound bed into the dressing where they are then killed, and/or prevent migration of microbes from the external environment through the dressing so that they are killed before reaching the wound site.

The present invention provides a multi-layer wound dressing comprising:
at least one interior layer, the at least one interior layer containing PHMB or a PHMB derivative comprising a polymeric biguanide that is cationic, displaces divalent cations from the wall and membrane of bacteria and bring about disruption of the lipid bilayer in an amount of at least about 3,000 ppm; and
a first exterior layer, wherein the at least one interior layer is at a location within the dressing that is not intended to be directly applied to the surface of the skin or to a wound bed, and the exterior layer is at a location that (i) has a surface adapted to contact the surface of the skin or the wound bed and an opposing surface in contact with an interior layer, or (ii) a surface that faces away from the surface of the skin or wound bed and is exposed to the external environment, as well as an opposing surface for contact with an interior layer or surface of the dressing, characterized in that the first exterior layer contains PHMB or a PHMB derivative in an amount less than the amount of PHMB or PHMB derivative contained in the at least one interior layer.

A wound dressing according to the present invention can alternatively comprise a multi-layer wound dressing comprising: at least one interior layer, the at least one interior layer containing PHMB or a PHMB derivative in an amount of at least about 30,000 ppm; a first outer layer, the first exterior layer containing PHMB or a PHMB derivative in an amount of at least 10,000ppm; and a second exterior layer, the second outer layer containing PHMB or a PHMB derivative in an amount of at least 10,000ppm.

A wound dressing according to a further alternative optional aspect of the present invention may be a multi-layer wound dressing comprising: at least one interior layer; and at least one exterior layer; wherein at least one of the interior and exterior layers contains PHMB or a PHMB derivative, and the other layer comprises a chelating agent.

According to the present invention, there can also be provided a wound dressing in to form of a multi-layer wound dressing comprising: at least one interior layer; and at least one exterior layer; wherein at least one of the interior and exterior layers contains PHMB or a PHMB derivative, zinc or a zinc-containing agent, or both.

A wound dressing formed according to another alternative aspect of the present invention can include a multi-layer wound dressing comprising: at least one interior layer comprising a woven, non-woven, foam, gel, film, or a mixture thereof, the at least one interior layer containing at least one of PHMB or a PHMB derivative and zinc or a zinc-containing compound; and at least one exterior layer comprising calcium alginate, PHMB or a PHMB derivative, and a zinc-containing agent.

The present invention also contemplates a multi-layer wound dressing comprising: at least one interior layer containing an antimicrobial agent; and at least one exterior layer containing a cell-signaling agent.

A wound dressing formed according to yet another alternative configuration can comprise a wound dressing comprising a layer of cellulose or cellulose-based material containing at least about 10,000ppm of PHMB or PHMB derivative.

"Containing" or "contains" is to be broadly construed to mean that the one or more layers themselves and/or the materials making up the layers are impregnated with, and/or have coatings/treatments of other materials/agents applied thereto. The materials/agents may be applied to all or a portion of the layers or materials forming the layers. Finally, the term encompasses all methods or techniques of impregnation and/or coating/treatment, regardless of the state of the materials/agents being applied thereto (e.g., solid, liquid, gas, plasma, etc.). The added materials/agents can be applied during manufacture, or subsequent thereto (e.g., by the user/consumer prior to application of the one or more layers to the wound site). The terms also do not preclude the presence of other substances or materials, and should be construed as being equivalent to the term "comprising" in this regard.

As used herein, "PHMB" refers to polyhexamethylene biguanide, and "PHMB derivative" refers to polymeric biguanides that are cationic, displace divalent cations from the wall and membrane of bacteria and bring about disruption of the lipid bilayer. PHMB derivatives include, but are not limited to polyethylene hexamethylene biguanide (PEHMB) chlorohexadine glucomate, biodegradable PHMB , and other members of the biguanide family of antimicrobials.

As used herein, "interior layer" refers to a location within the dressing that is not intended to be directly applied to the surface of the skin or to a wound bed. As used herein, "exterior layer" refers to a location that (i) has a surface adapted to contact the surface of the skin or the wound bed and an opposing surface in contact with an interior layer, or (ii) a surface that faces away from the surface of the skin or wound bed and is exposed to the external environment, as well as an opposing surface for contact with an interior layer or surface of the dressing.

As used herein, "parts per million" or "ppm" refers to the amount of agent or substance contained within the dressing as determined by extracting the agent or substance out of the dressing material, and measuring the weight of extracted material versus the dry weight of the dressing material. For example, extraction can be conducted by soaking the dressing loaded with the agent or substance in 0.9% NaCl in water by weight (Isotonic saline) or 1M acetic acid overnight at a temperature of approximately 56°C. The agent or substance in the resulting solution was identified via UV spectrophotometer or HPLC. This value is quantified by plotting the resulting peak against the standard dilution curve. The resulting loading level of agent or substance can then be calculated on a "ppm" basis.

As used herein "microbially-derived" or "microbially-derived" refers to cellulose or cellulose-based material formed consistent with the teachings of U.S. Patent Application Publication Nos. 2004/0028722, 2004/0142019, and 2005/0019380, and which is distinguished from plant-derived cellulose.

### BRIEF DESCRIPTION OF THE DRAWINGS

FIG. 1 is a schematic illustration of an exemplary embodiment of a wound dressing of the present invention.

FIG. 2 is a schematic cross-sectional illustration, taken along lines 2-2 of Figure 1 and can represent alternative embodiments of a wound dressing of the present invention.

### DETAILED DESCRIPTION

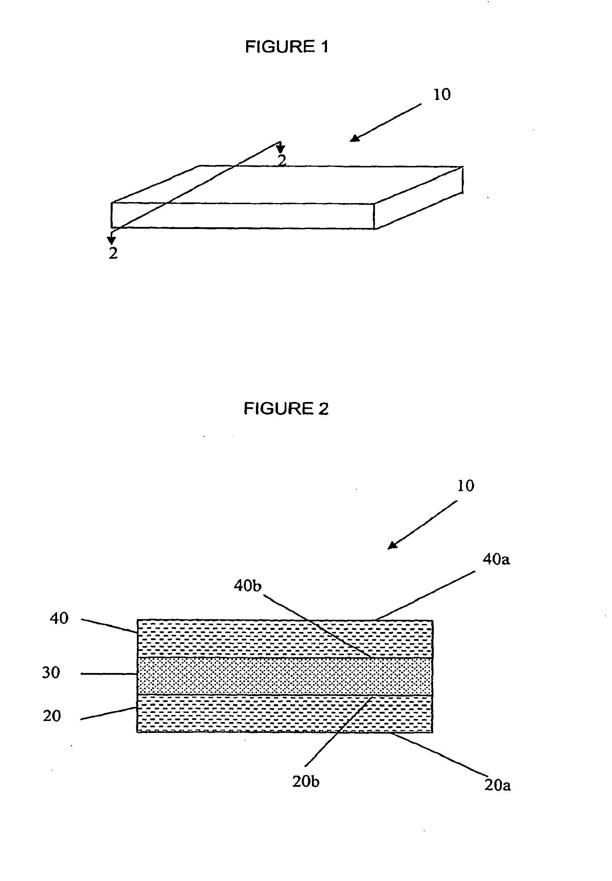

Figures 1-2 may be referred to in order to facilitate the following discussion. A wound dressing 10 formed according to the principles of the present invention can be generally formed from one or more discrete layers (e.g., 20, 30, 40). When composed of multiple layers, the dressing 10 includes at least one interior layer 30 as well as one or more exterior layer(s) 20, 40. The exterior layer(s) being characterized by at least one surface 20a adapted for contact with the surface of the skin of a wearer, or a wound bed, and an opposing surface 20b contacting an interior layer, or at least one surface 40a facing away from the surface of the skin or wound bed and exposed to the surrounding environment as well as an opposing surface 40b contacting an interior layer. The interior layer(s) 30 lack either a surface for contact with the skin surface or wound bed, or which is both exposed to the external environment and in contact with an interior layer.

While the illustrated embodiment includes one interior layer 30 and two exterior layers 20, 40, it should be understood that the invention is not limited to such a construction. Any suitable number of layers may be present. According to certain exemplary embodiments, the dressing 10 can be in the form of a single layer. Alternatively, the dressing 10 can have only two layers. According to further alternative constructions, the dressing 10 can have more than three layers. For example, the dressing can have 4, 5, 6, 7, 8 or more layers.

According to the present invention, the anti-microbial agent(s) and/or other components or agents identified herein can be added to the various interior and/or exterior layers of the dressing in any suitable manner. For example, the agent(s) can be sprayed onto the dressing layer(s), or the dressing layer(s) can be soaked or dipped in a solution containing the agent(s), then dried. The agent(s) can optionally be combined with the various interior and/or exterior layers of the dressing so as to render them releasable therefrom (e.g., so as to migrate out of the layer(s), toward, and into the wound bed). For example, the dressing can be moistened with a predetermined amount of isotonic saline (e.g., 0.9% Na) or sodium citrate, then combined with an antimicrobial, which can be contained in the saline or citrate medium, or added sequentially thereto.

In addition, due to the optional absorbent characteristics of the dressing, microbes are absorbed within the layer(s) of the dressing and killed by the antimicrobial and/or other agent(s) contained therein and prevented from passing through the dressing. Thus, it can be advantageous to combine the agent(s) with the dressing material in a manner that prevents substantial amounts of agent(s) from leaving the dressing. For example, the dressing can be cured at a specific pH level (e.g., pH = 7 +/- 0.4). The agent(s) will only be released in large amounts when the pH of the dressing reduces around 5 or less, which is not a typical pH associated with wound exudate.

The interior layer(s) 30 may be substantially hydrophilic, while one or more of the outer layers 20,40 may be substantially hydrophobic. The term "substantially hydrophilic" describes the function of the interior layer material. It also distinguishes the interior layer material over the function of the "substantially hydrophobic" exterior layer material, which can act to provide an anti-microbial barrier property and attenuates or reduces the release of anti-microbial agent from the interior layer(s) 30 away from the dressing. Retention of anti-microbial agent within the interior layer also lowers the bioburden, i.e., the growth and number of cells, within the dressing during use. The various layers of the dressing material can be provided with the desired hydrophilic or hydrophobic properties in accordance with any suitable known manner. Exemplary constructions and techniques are described in U.S. Patent Application Publication No. 2004/0082925.

Each of the one or more layers can be formed from any suitable material and/or construction. For example, the one or more layers can be formed from a material that is fibrous, film-like, gel, or combinations thereof. With respect to fibrous materials, they can be woven or nonwoven materials. The fibers can be selected from natural fibers, synthetic fibers, and combinations of the two. By way of non-limiting example, suitable materials which can be utilized to form the one or more layers of the present invention may include: cellulose, non-microbially derived cellulose, cellulose acetate, oxycellulose, alginates, cotton, polypropylene, polyvinyl alcohol, rayon, nylon, acrylic, polyester, polyurethane, hydrogels, hydrocolloids and combinations thereof.

According to one optional embodiment, at least one exterior layer 20, 40 can be constructed to be dissolvable or absorbable. Accordingly, one or more layers of the dressing may comprise a bioabsorbable material such as polyglycolic acid, polylactic acid, collagen, chitin, keratin, an alginate, guar gum, locust bean gum or derivatives or mixtures thereof. The layer also may comprise a bioabsorbable polymer formed by chemically modifying a natural substance, for example, oxidized cellulose or chitosan or a cross-linked hyaluronic acid gel.

A wound dressing of the present invention may include one or more anti-microbial agents. A number of alternative anti-microbial agents are possible. Suitable anti-microbial agents include, but are not limited to, a chlorohexidine, a chlorohexadine salt, a triclosan, a polymoxin, a tetracycline, an amino glycoside (e.g., gentamicin or Tobramycin™), a rifampicin, a bacitracin, an erythromycin, a neomycin, a chloramphenicol, a miconazole, a quinolone, a penicillin, a nonoxynol 9, a fusidic acid, a cephalosporin, a mupirocin, a metronidazole, a secropin, a protegrin, a bacteriolcin, a defensin, a nitrofurazone, a mafenide, a acyclovir, a vanocmycin, a clindamycin, a lincomycin, a sulfonamide, a norfloxacin, a pefloxacin, a nalidizic acid, an oxalic acid, an enoxacin acid, a ciprofloxacin, a biguanide (e.g., PHMB), combinations thereof and the like. In certain embodiments the anti-microbial agent can comprise polyhexamethylene biguanide (PHMB) or a derivative thereof. The antimicrobial agent can be present in the dressing at any suitable level which provides an adequate an adequate anti-microbial effect. For example, suitable concentration levels include, but are not limited to, 2,000 ppm, 2,500 ppm, 3,000 ppm, 3,500 ppm, 5,000 ppm, 10,000 ppm, 13,000 ppm, 30,000 ppm, and combinations and/or gradients thereof. According to one optional embodiment, the dressing contains PHMB or a PHMB derivative present in any of the above-listed amounts.

A wound dressing of the present invention may further include a chelating agent, as an additional component or as a full or partial substitute for any of the above. Any suitable chelating agent may be utilized. By way of non-limiting example, chelating agents such as ethylenediaminetetraacetic acid (EDTA), variations of EDTA such as, for example, disodium EDTA or tetrasodium EDTA, combinations thereof and the like, are contemplated. Other chelating agents such as citrate and heprin are also contemplated by the present invention. Chelating agents can heighten the susceptibility of bacteria and other organisms to the antiseptic effects of another anti-microbial agent, thereby rendering the wound dressing more effective in combating and/or preventing infection. Generally, chelating agents advantageously (i) are non-thrombogenic; (ii) are more active in an acidic environment; (iii) re-sensitize microbes to the effects of other antimicrobial agents; (iv) provide a debriding effect and (v) remove ionic attractions necessary to form/sustain biofilms. This aspect of the present invention can advantageously avoid problems caused by the potentially irritating effects of certain anti-microbial agents, such as PHMB, especially when applied to the skin at higher concentration levels. The chelating agent can be present at any suitable concentration. For example, the chelating agent can be present in amounts on the order of about 0.05 to about 1.0% by weight.

As an additional component, or as a full or partial substitute for one or more of the above-mentioned anti-microbial agents and/or chelating agents, a wound dressing formed according to the principles of the present invention may include one or more additional anti-microbial agents. By way of non-limiting example, suitable additional anti-microbial agents include, but are not limited to: polyethylene hexamethylene biguanide (PEHMB), silver, copper, and combinations thereof. The one or more additional anti-microbial agents can be present at any suitable concentration level. For example, the dressing can contain 1 to 3% by weight silver of the additional anti-microbial agent(s).

As an additional component, or as a full or partial substitute for one or more of the above-mentioned anti-microbial agents, chelating agents and/or additional anti-microbial agents, the dressing may further include a zinc-containing agent. Suitable zinc-containing agents include, but are not limited to, zinc, zinc alginate, zinc bacitracin, zinc oxide, zinc phosphate, zinc aspartate, and combinations thereof. According to one optional embodiment of the present invention the zinc-containing agent includes zinc acetate, zinc butyrate, zinc citrate, zinc gluconate, zinc glycerate, zinc glycolate, zinc formate, zinc lactate, zinc picolinate, zinc propionate, zinc salicylate, zinc tartrate, zinc undecylenate, Zinc Stearate and combinations thereof. Zinc-containing agents can improve the rate of wound healing, thereby rendering the wound dressing more effective in combating and/or preventing infection, without the necessity of increasing the levels of anti-microbial agent contained therein. Combination with an aliginate provides moisture-absorption capabilities, and alginates help promote a moist wound healing environment. This aspect of the present invention advantageously avoids problems caused by the irritating effects of certain anti-microbial agents, such as PHMB, especially when applied to the skin that higher concentration levels.

As an additional component, or as a full or partial substitute for one or more of the above-mentioned anti-microbial agents, chelating agents, additional anti-microbial agents, and/or zinc-containing agents, the dressing may further include a cell-signaling agent. A cell-signaling agent provides a mechanism for communicating with the cell by electrical, chemical or biologic means that encourages cell growth or movement or receptive action in the direction of the signal. The signal may also deactivate the bacterial cells' defense mechanisms. According to this construction, bacterial growth is promoted in a preferred manner (i.e., away from the wound bed) which leads to an increased efficacy of the wound dressing.

Exemplary wound dressings can, of course, include additional active ingredients or agents such as, for example, a therapeutic agent, an organoleptic agent, a growth factor, an analgesic, a tissue scaffolding agent, a haemostatic agent, a protein inhibitor, collagen, enzymes, an anti-thrombogenic agent, an anesthetic, an anti-inflammatory agent, an anticancer agent, a vasodilation substance, a wound healing agent, an angiogenic agent, an angiostatic agent, an immune boosting agent, a skin sealing agent, an agent to induce directional bacterial growth, an agent to impart bactericidal or bacteriostatic activity, an electron transfer agent to destabilize or destroy the metabolic action of microbes and/or biofilm formation, combinations thereof and the like. These agents can be present in the dressing in any suitable amount, such as about 0.05 to about 1.0% by weight. Release of active agents may be triggered by a variety of means, such as, for example, an electric field or signal, temperature, time, pressure, moisture, light (e.g., ultra-violet light), ultrasound energy, sonication, combinations thereof and the like. By way of non-limiting example, the additional agent can comprise silver or compounds thereof.

According to the present invention, any of the above-mentioned components or agents may be combined directly with the material forming the one or more layers of the wound dressing in any conventional manner. Alternatively, any of the above-mentioned agents may be contained, and subsequently released, by a delivery agent. Any suitable delivery agent can be utilized. By way of non-limiting example, suitable delivery agents include: a hydrogel, phosphate glass, powdered carrier, or a film carrier.

The anti-microbial, chelating agent, additional anti-microbial agent, zinc-containing agent, cell-signalling agent and/or or other agent mentioned above can optionally be printed or otherwise applied to one or more layers of a wound dressing to provide a desired concentration or concentration gradient of one or more of these agents on and/or within the dressing. For example, one or more of the agents can be applied separately, or in combination, in a specific pattern corresponding to the wound area, for the purpose of optimizing the anti-microbial and wound healing effects.

Wound dressings formed according to the present invention can be provided in numerous configurations, having a number of different combinations of features. In the discussion that follows, any of the above-mentioned agents or additives can optionally be included in the illustrative configurations discussed below, unless otherwise indicated.

According to one possible configuration of the present invention, a wound dressing is provided which comprises one or more layers containing at least one anti-microbial agent and at least one chelating agent. According to one optional configuration, all layers of the wound dressing may contain a combination of anti-microbial agent and chelating agent. The anti-microbial agent can be present in amounts of about 2500 to about 30,000 ppm. The chelating agent can be present in amounts of about 1,000 to about 10,000 ppm.

According to another alternative modification of the above multi-layer configuration, the anti-microbial agent and the chelating agent can be separately contained in different layers of the wound dressing. Thus, for example, a wound dressing can be formed with at least one interior layer (e.g., 30), and at least one exterior layer (e.g., 20, 40). The anti-microbial agent can be contained in the interior layer 30, which is not in direct contact with the skin or wound, and the chelating agent can be provided in one or more exterior layer(s) 20, 40. According to one embodiment, the interior layer 30 contains about 30,000 ppm PHMB or a derivative thereof and one or more exterior layers 20, 40 contain about 5,000 ppm EDTA. According to an further optional embodiment, the interior layer(s) 30 can be constructed so as to prevent the escape of a significant amount of anti-microbial agent therefrom, while at least one of the exterior layers can be constructed so as to permit the migration of the chelating agent into the surface of the skin or wound bed.

As an optional modification of the above, the chelating agent can be provided in the interior layer 30, and the anti-microbial agent provided in one or more of the exterior layers 20, 40.

According to a further alternative construction, the.wound dressing 10 is formed from a plurality of different layers and materials containing agents to enhance performance. A cell-signaling agent of the type described above can be provided in the dressing between the wound bed and another dressing layer which is treated with one or more of the anti-microbial agents identified herein. For example, an exterior layer 20 can be provided which contains the cell-signaling agent, and an interior layer 30 can be provided that contains one or more antimicrobial agent(s) including PHMB or a derivative thereof. According to this construction, bacteria would need to cross the anti-microbial agent to reach the signaling mechanism, and bacterial growth is promoted in a preferred manner (i.e., away from the wound bed) which leads to an increased efficacy of the wound dressing.

According to one possible alterative configuration of the present invention, a wound dressing 10 can be provided which comprises one or more layers containing at least one anti-microbial agent and/or at least one zinc-containing agent.

According to one optional modification, all layers of the wound dressing may contain a combination of the anti-microbial agent and zinc-containing agent.

According to another alternative modification of the above configuration, the wound dressing comprises a plurality of layers and the anti-microbial agent and the zinc-containing agent can be separately contained in different layers of the wound dressing. As one possible example of this configuration, at least one of the layers contains both an anti-microbial agent and a zinc-containing agent, while other layer(s) separately contain the anti-microbial agent or zinc-containing agent.

For example, the interior layer(s) 30, which is not in direct contact with the skin or wound, contains PHMB or a derivative thereof, and the zinc-containing agent can be provided in one or more of the outer layers 20, 40.

According to an alternative construction, at least one interior layer 30 and at least one exterior layer 20, 40 each contain a combination of PHMB or a derivative thereof and zinc-containing agent. The layers may have different concentrations of the PHMB or derivative thereof and/or zinc-containing agent. According to one embodiment, layer 30 contains about 30,000 ppm PHMB, and layers 20, 40 each contain about 0.1 to about 3.0% zinc alginate by weight.

According to a further alternative construction, at least one interior layer 30 contains a combination of PHMB or a derivative thereof and zinc, and at least one exterior layer 20, 40 can contain a zinc-containing agent.

According to yet another alternative configuration, at least one interior layer 30 contains a combination of PHMB or a derivative thereof and a zinc-containing agent, and at least one exterior layer 20, 40 contains a combination of calcium alginate, PHMB or a derivative thereof, and a zinc-containing agent.

The present invention is also directed to the construction of multi-layer dressings that have layers with different concentrations of anti-microbial agent.

According to one optional configuration, a dressing can be constructed such that it is provided with at least one interior layer and at least one exterior layer both which contain an anti-microbial agent in different amounts. Specifically, at least one interior layer contains a relatively higher concentration of anti-microbial agent and at least one of the exterior layers contained in the dressing.

According to one optional embodiment, the dressing contains at least one interior layer 30 which contains PHMB or a PHMB derivative in an amount of at least 3,000 ppm, and at least one exterior layer which contains PHMB or a PHMB derivative in an amount which is less than 3,000 ppm. According to various optional modifications of this construction, the at least one interior layer 30 can contain PHMB or a PHMB derivative in amounts of at least 3,500 ppm, at least 5,000 ppm, at least 10,000 ppm, at least 13,000 ppm, or at least 30,000 ppm, while the at least one exterior layer 20, 40 of the dressing also contains PHMB or a PHMB derivative in an amount which is less than the amount contained in the at least one interior layer 30.

According to one optional, and more specific embodiment of the above described construction, a dressing can be provided having at least one interior layer 30 which contains at least about 13,000 ppm of PHMB or a PHMB derivative, and at least one exterior layer 20, 40 which contains at least about 2,000 ppm PHMB or PHMB derivative. According to one variation of this embodiment, the dressing comprises two exterior layers 20, 40, each containing at least about 2,000 ppm PHMB or PHMB derivative.

According to another optional, and more specific embodiment of the above described construction, addressing can be provided having at least one interior layer 30 which contains at least about 30,000 ppm PHMB or PHMB derivative, and at least one exterior layer 20, 40 which contains at least about 10,000 ppm PHMB or PHMB derivative. According to one variation of this embodiment, the dressing comprises two exterior layers 20, 40, each containing at least about 10,000 ppm PHMB or PHMB derivative.

According to one optional embodiment, the at least one interior layer 30 can be constructed to prevent elution of substantial amounts of PHMB or PHMB derivative into adjacent layers of the dressing and/or into the skin or wound bed. According to a further optional embodiment of the above, the at least one exterior layer 20, 40 can be constructed so as to permit PHMB or PHMB derivative to elute into the skin or wound bed.

The present invention also contemplates a wound dressing comprising a layer formed primarily from a cellulose or cellulose-based material which contains PHMB or a PHMB derivative. The cellulose or cellulose-based dressing material can comprise at least about 50% cellulose material. According to one optional embodiment the dressing material comprises 100% cellulose material. The cellulose material may optionally comprise rayon. According to one optional embodiment, dressing can include a layer of cellulose or cellulose-based material which contains at least 5000 ppm PHMB or PHMB derivative. According to certain optional modifications of this construction, a layer of cellulose or cellulose-based material can contain PHMB or PHMB derivative in amounts of at least about 10,000 ppm, at least about 13,000 ppm, or at least about 30,000 ppm. According to a further optional modification of this embodiment, the cellulose or cellulose-based material is not microbially-derived. According to yet another optional modification of this construction, the dressing can be formed of a single layer, without any additional layers contained therein.

All numbers expressing quantities of ingredients, constituents, reaction conditions, and so forth used in the specification are to be understood as being modified in all instances by the term "about." Notwithstanding that the numerical ranges and parameters setting forth, the broad scope of the subject matter presented herein are approximations, the numerical values set forth are indicated as precisely as possible. Any numerical value, however, inherently contains certain errors necessarily resulting from the standard deviation found in their respective measurement techniques.

Although the present invention has been described in connection with preferred embodiments thereof, it will be appreciated by those skilled in the art that additions, deletions, modifications, and substitutions not specifically described may be made without departure from the scope of the invention as defined in the appended claims.

In one embodiment, the dressings include at least one interior layer that comprises at least about 13,000 ppm of PHMB or PHMB derivative.

In another embodiment, the dressings include those wherein at least one interior layer that is constructed to prevent elution of substantial amounts of PHMB or PHMB derivative into adjacent layers of the wound dressing.

In another embodiment, the dressings include those wherein at least one of the first and second exterior layers is constructed to elude PHMB or PHMB derivative into a wound bed when applied to a wound surface.

In another embodiment, the dressings include those wherein at least one of the first and second outer layers is dissolvable or absorbable.

In another embodiment, the dressings include those wherein one or more of the interior and first and second exterior layers are formed from a material comprising: cotton, polypropylene, polyvinyl alcohol, polyester, rayon, polyurethane, acrylic, cellulose, cellulose acetate, alginate, and combinations thereof.

In another embodiment, the dressings include those wherein: at least one interior layer contains PHMB or a PHMB derivative in an amount of at least about 30,000 ppm; a first outer layer contains PHMB or a PHMB derivative in an amount of at least 10,000ppm; and a second outer layer contains PHMB or a PHMB derivative in an amount of at least 10,000ppm.

In another embodiment, all of the dressings herein further comprise a therapeutic agent, an organoleptic agent, a growth factor, an analgesic, a tissue scaffolding agent, a haemostatic agent, a protein inhibitor, collagen, enzymes, an anti-thrombogenic agent, an anesthetic, an anti-inflammatory agent, an anticancer agent, a vasodilation substance, a wound healing agent, an angiogenic agent, an angiostatic agent, an immune boosting agent, a skin sealing agent, an agent to induce directional bacterial growth, an agent to impart bacteriacidal or bacteriostatic activity, an electron transfer agent to destabilize or destroy the metabolic action of microbes and/or biofilm formation, combinations thereof.

In another embodiment, the dressings include those wherein one or more of the interior and exterior layers are formed from a material comprising: cotton, polypropylene, polyvinyl alcohol, polyester, rayon, polyurethane, acrylic, cellulose, cellulose acetate, alginate, and hydrogels, hydrocolloids and combinations thereof.

In another embodiment, the dressings include those wherein at least one interior layer comprises PHMB or a PHMB derivative, and at least one exterior layer comprises a chelating agent.

In another embodiment, the dressings include those wherein at least one interior layer comprises a chelating agent, and at least one exterior layer comprises PHMB or a PHMB derivative.

In another embodiment, the dressings include those wherein the chelating agent comprises EDTA, heparin, or citrate.

In another embodiment, the dressings include those wherein the chelating agent is releasably contained by a hydrogel, a starch film or powder, or dissolvable beads.

In another embodiment, the dressings include those wherein the PHMB or PHMB derivative is present in an amount of about 2,500 to about 30,000 ppm and the chelating agent is present in an amount of about 1,000 to about 10,000 ppm.

In another embodiment, the dressings include those wherein one or more of the interior and exterior layers are formed from a material comprising: cotton, polypropylene, polyvinyl alcohol, polyester, rayon, polyurethane, acrylic, cellulose, cellulose acetate, alginate, hydrogels, hydrocolloids and combinations thereof

In another embodiment, the dressings include those wherein at least one interior layer comprises PHMB or a PHMB derivative, and at least one exterior layer comprises a zinc-containing agent.

In another embodiment, the dressings include those wherein at least one interior layer and at least one exterior layer both comprise PHMB or a PHMB derivative as well as a zinc-containing agent.

In another embodiment, the dressings include those wherein: at least one interior layer comprises at least 2,500 to about 30,000 ppm of PHMB or a PHMB derivative and about 1.0-3.0% by weight a zinc-containing agent; and at least one exterior layer comprises at least about 1,500 to about 3,500 ppm of PHMB or a PHMB derivative and about 0.1 to 1.0% by weight of a zinc-containing agent.

In another embodiment, the dressings include those wherein: at least one interior layer comprises at least about 10,000 ppm of PHMB or a PHMB derivative and at least about 30,000 ppm of a zinc-containing agent; and at least one exterior layer comprises at least about 20,000 ppm of PHMB or a PHMB derivative and at least about 40,000 ppm of a zinc-containing agent.

In another embodiment, the dressings include those wherein: at least one interior layer comprises at least about 50,000 ppm zinc-containing agent; and at least one exterior layer comprises at least about 60,000 ppm zinc-containing agent.

In another embodiment, the dressings include those wherein at least one interior layer comprises PHMB or a PHMB derivative as well as a zinc-containing agent, and at least one exterior layer comprises a zinc-containing agent.

In another embodiment, the dressings include those wherein the zinc-containing agent comprises: zinc, zinc alginate, zinc bacitracin, zinc oxide, zinc phosphate, or zinc aspartate.

In another embodiment, the dressings include those wherein at least one of a PHMB or PHMB derivative and a zinc-containing agent are printed onto one or more of the interior and exterior layers.

In another embodiment, the dressings include those wherein: at least one interior layer comprises a woven, non-woven, foam, gel, film, or a mixture thereof, at least one interior layer further containing at least one of PHMB or a PHMB derivative, and a zinc-containing agent; and at least one exterior layer contains calcium alginate, PHMB or a PHMB derivative, and a zinc-containing agent.

In another embodiment, the dressings include those wherein at least one exterior layer comprises at least about 1,500 to about 3,500 ppm of PHMB or PHMB derivative and at least about 0.1 to about 0.1 to 1.0% by weight zinc-containing agent.

In another embodiment, the dressings herein may further comprise a cell-signaling agent.

In another embodiment, the dressings include those wherein at least one interior layer comprises at least about 13,000 ppm of antimicrobial agent, and at least one exterior layer comprises at least about 2,000 ppm of cell-signaling agent.

In another embodiment, the dressings include those wherein the cellulose-based material comprises oxycellulose.

## Claims

1. A multi-layer wound dressing (10) comprising:
at least one interior layer (30), the at least one interior layer (30) containing PHMB or a PHMB derivative comprising a polymeric biguanide that is cationic, displaces divalent cations from the wall and membrane of bacteria and bring about disruption of the lipid bilayer in an amount of at least about 3,000 ppm; and
a first exterior layer (20), wherein the at least one interior layer (30) is at a location within the dressing (10) that is not intended to be directly applied to the surface of the skin or to a wound bed, and the exterior layer (20) is at a location that (i) has a surface (20a) adapted to contact the surface of the skin or the wound bed and an opposing surface (20b) in contact with an interior layer (30), or (ii) a surface (40a) that faces away from the surface of the skin or wound bed and is exposed to the external environment, as well as an opposing surface (40b) for contact with an interior layer (30) or surface of the dressing, **characterized in that** the first exterior layer (20) contains PHMB or a PHMB derivative in an amount less than the amount of PHMB or PHMB derivative contained in the at least one interior layer (30).

2. The dressing (10) of claim 1, wherein the at least one interior layer (30) comprises at least about 3,500 ppm of PHMB or PHMB derivative.

3. The dressing (10) of claim 2, wherein the at least one interior layer (30) comprises at least about 5,000 ppm of PHMB or PHMB derivative.

4. The dressing (10) of claim 3, wherein the at least one interior layer (30) comprises at least about 10,000 ppm of PHMB or PHMB derivative.

5. The dressing (10) of claim 4, wherein the at least one interior layer (30) comprises at least about 30,000 ppm of PHMB or PHMB derivative.

6. The dressing (10) of claim 1, further comprising:
a second exterior layer (40), the second exterior layer (40) containing PHMB or PHMB derivative in an amount less than the amount of PHMB or PHMB derivative contained in the at least one interior layer (30).

7. The dressing (10) of claim 6, wherein the at least one interior (30) layer comprises at least about 13,000 ppm of PHMB or PHMB derivative; and
wherein the first and second exterior layers (20,40) each contain at least about 2,000 ppm of PHMB or PHMB derivative.

8. The dressing (10) of claim 1, further comprising a therapeutic agent, an organoleptic agent, a growth factor, an analgesic, a tissue scaffolding agent, a haemostatic agent, a protein inhibitor, collagen, enzymes, an anti-thrombogenic agent, an anesthetic, an anti-inflammatory agent, an anticancer agent, a vasodilation substance, a wound healing agent, an angiogenic agent, an angiostatic agent, an immune boosting agent, a skin sealing agent, an agent to induce directional bacterial growth, an agent to impart bacteriacidal or bacteriostatic activity, an electron transfer agent to destabilize or destroy the metabolic action of microbes and/or biofilm formation, combinations thereof.

9. The dressing (10) of claim 1, wherein at least one of the interior and exterior layers (20,30,40) comprises PHMB or a PHMB derivative, and the other layer comprises a chelating agent.

10. The dressing (10) of claim 1, wherein at least one of the interior and exterior layers (20,30,40) comprises PHMB or a PHMB derivative, and the other layer contains a zinc-containing agent.

## Patentansprüche

1. Mehrschicht-Wundverband (10), umfassend:
wenigstens eine innere Schicht (30), wobei die wenigstens eine innere Schicht (30) PHMB oder ein PHMB-Derivat enthält, umfassend ein polymeres Biguanid, das kationisch ist, zweiwertige Kationen von der Wand und der Membran von Bakterien verdrängt und in einer Menge von wenigstens etwa 3.000 ppm Zerreißen der Lipid-Doppelschicht bewirkt; und
eine erste äußere Schicht (20), wobei die wenigstens eine innere Schicht (30) an einer Position innerhalb des Verbands (10) angeordnet ist, die nicht zum direkten Aufbringen auf die Oberfläche der Haut oder auf ein Wundbett vorgesehen ist, und die äußere Schicht (20) an einer Position angeordnet ist, die (i) eine Oberfläche (20a) aufweist, die für den Kontakt mit der Oberfläche der Haut oder dem Wundbett ausgelegt ist, und eine gegenüberliegende Oberfläche (20b) in Kontakt mit einer inneren Schicht (30), oder (ii) eine Oberfläche (40a), die von der Oberfläche der Haut oder des Wundbetts weg gerichtet ist und gegenüber der äußeren Umgebung exponiert ist, sowie eine gegenüberliegende Oberfläche (40b) für den Kontakt mit einer inneren Schicht (30) oder Oberfläche des Verbands, **dadurch gekennzeichnet, dass** die erste äußere Schicht (20) PHMB oder ein PHMB-Derivat in einer Menge enthält, die kleiner ist als die Menge an PHMB oder PHMB-Derivat, die in der wenigstens einen inneren Schicht (30) enthalten ist.

2. Verband (10) gemäß Anspruch 1, wobei die wenigstens eine innere Schicht (30) wenigstens etwa 3.500 ppm an PHMB oder PHMB-Derivat enthält.

3. Verband (10) gemäß Anspruch 2, wobei die wenigstens eine innere Schicht (30) wenigstens etwa 5.000 ppm an PHMB oder PHMB-Derivat enthält.

4. Verband (10) gemäß Anspruch 3, wobei die wenigstens eine innere Schicht (30) wenigstens etwa 10.000 ppm an PHMB oder PHMB-Derivat enthält.

5. Verband (10) gemäß Anspruch 4, wobei die wenigstens eine innere Schicht (30) wenigstens etwa 30.000 ppm an PHMB oder PHMB-Derivat enthält.

6. Verband (10) gemäß Anspruch 1, ferner umfassend:
eine zweite äußere Schicht (40), wobei die zweite äußere Schicht (40) PHMB oder PHMB-Derivat in einer Menge enthält, die kleiner ist als die Menge an PHMB oder PHMB-Derivat, die in der wenigstens einen inneren Schicht (30) enthalten ist.

7. Verband (10) gemäß Anspruch 6, wobei die wenigstens eine innere Schicht (30) wenigstens etwa 13.000 ppm an PHMB oder PHMB-Derivat umfasst; und
wobei die erste und die zweite äußere Schicht (20, 40) jeweils wenigstens etwa 2.000 ppm an PHMB oder PHMB-Derivat enthält.

8. Verband (10) gemäß Anspruch 1, ferner umfassend ein therapeutisches Mittel, ein organoleptisches Mittel, einen Wachstumsfaktor, ein Analgetikum, ein Gewebegerüstmittel, ein hämostatisches Mittel, einen Proteinhemmer, Collagen, Enzyme, ein antithrombogenes Mittel, ein Anästhetikum, ein entzündungshemmendes Mittel, ein Antikrebsmittel, einen vasodilatorischen Stoff, ein Wundheilungsmittel, ein angiogenes Mittel, ein angiostatisches Mittel, ein immunförderndes Mittel, ein Hautheilungsmittel, ein Mittel zum Induzieren von gerichtetem Bakterienwachstum, ein Mittel zum Verleihen von bakterizider oder bakteriostatischer Wirksamkeit, ein Elektronenübertragungsmittel zum Destabilisieren oder Zerstören der metabolischen Wirkung von Mikroben und/oder der Biofilmentstehung, Kombinationen davon.

9. Verband (10) gemäß Anspruch 1, wobei wenigstens eine von der inneren und den äußeren Schichten (20, 30, 40) PHMB oder ein PHMB-Derivat umfasst und die andere Schicht ein chelatbildendes Mittel umfasst.

10. Verband (10) gemäß Anspruch 1, wobei wenigstens eine von der inneren und den äußeren Schichten (20, 30, 40) PHMB oder ein PHMB-Derivat umfasst und die andere Schicht ein Zinkenthaltendes Mittel enthält.

## Revendications

1. Pansement multicouche (10), comprenant :
au moins une couche intérieure (30), ladite au moins une couche intérieure (30) contenant du PHMB ou un dérivé de PHMB comprenant un biguanide polymère qui est cationique, déplace les cations divalents de la paroi et de la membrane des bactéries et provoque la rupture de la double couche lipidique, en une quantité d'au moins environ 3000 ppm ; et
une première couche extérieure (20), ladite au moins une couche intérieure (30) étant à un emplacement à l'intérieur du pansement (10) qui n'est pas destiné à être appliqué directement à la surface de la peau ou au lit d'une plaie, et la couche extérieure (20) étant située à un emplacement qui (i) a une surface (20a) adaptée au contact avec la surface de la peau ou le lit de la plaie et une surface opposée (20b) en contact avec une couche intérieure (30), ou (ii) une surface (40a) qui est à l'opposé de la surface de la peau ou du lit de la plaie et qui est exposée à l'environnement extérieur, ainsi qu'une surface opposée (40b) pour le contact avec une couche intérieure (30) ou surface du pansement, **caractérisé en ce que** la première couche extérieure (20) contient du PHMB ou un dérivé de PHMB en une quantité inférieure à la quantité de dérivé de PHMB présente dans ladite au moins une couche intérieure (30).

2. Pansement (10) suivant la revendication 1, dans lequel ladite au moins une couche intérieure (30) comprend au moins environ 3500 ppm de PHMB ou de dérivé de PHMB.

3. Pansement (10) suivant la revendication 2, dans lequel ladite au moins une couche intérieure (30) comprend au moins environ 5000 ppm de PHMB ou de dérivé de PHMB.

4. Pansement (10) suivant la revendication 3, dans lequel ladite au moins une couche intérieure (30) comprend au moins environ 10 000 ppm de PHMB ou de dérivé de PHMB.

5. Pansement (10) suivant la revendication 4, dans lequel ladite au moins une couche intérieure (30) comprend au moins environ 30 000 ppm de PHMB ou de dérivé de PHMB.

6. Pansement (10) suivant la revendication 1, comprenant en outre :
une seconde couche extérieure (40), la seconde couche extérieure (40) contenant du PHMB ou un dérivé de PHMB en une quantité inférieure à la quantité de PHMB ou de dérivé de PHMB présente dans ladite au moins une couche intérieure (30).

7. Pansement (10) suivant la revendication 6, dans lequel ladite au moins une couche intérieure (30) comprend au moins environ 13 000 ppm de PHMB ou de dérivé de PHMB ; et
dans lequel les première et seconde couches extérieures (20, 40) contiennent chacune au moins environ 2000 ppm de PHMB ou de dérivé de PHMB.

8. Pansement (10) suivant la revendication 1, comprenant en outre un agent thérapeutique, un agent organoleptique, un facteur de croissance, un analgésique, un agent de support tissulaire, un hémostatique, un inhibiteur de protéines, du collagène, des enzymes, un agent anti-thrombogène, un anesthésique, un agent anti-inflammatoire, un agent anti-cancéreux, une substance vasodilatatrice, un agent cicatrisant, un agent angiogénique, un agent angiostatique, un stimulateur immunitaire, un agent d'occlusion cutanée, un agent pour induire une croissance bactérienne directionnelle, un agent pour conférer une activité bactéricide ou bactériostatique, un agent de transfert d'électrons pour déstabiliser ou détruire l'action métabolique de micro-organismes et/ou de formation de biofilm, ou leurs associations.

9. Pansement (10) suivant la revendication 1, dans lequel au moins une des couches intérieure et extérieure (20, 30, 40) comprend du PHMB ou un dérivé de PHMB, et l'autre couche comprend un agent chélatant.

10. Pansement (10) suivant la revendication 1, dans lequel au moins une des couches intérieure et extérieure (20, 30, 40) comprend du PHMB ou un dérivé de PHMB, et l'autre couche contient un agent contenant du zinc.
